# EUROPÄISCHE PATENTSCHRIFT

(11) **EP 2 870 968 B1**
(45) Veröffentlichungstag und Bekanntmachung des Hinweises auf die Patenterteilung: **10.01.2018**
(21) Anmeldenummer: 14192251.8
(22) Anmeldetag: 07.11.2014
(51) Int. Cl.: A61K 33/06, A61K 33/08, A61P 1/04

(54) **Behandlung entzündlicher Darmerkrankungenm mit Klinoptilolith**
Treatment of inflammatory bowel disease with clinoptilolith
Traitement de maladies inflammatoires de l'intestin avec du clinoptilolite

(30) Priorität: 08.11.2013 EP 13192094
(43) Veröffentlichungstag der Anmeldung: 13.05.2015
(73) Patentinhaber: Glock Health, Science and Research GmbH, 2232 Deutsch Wagram (AT)
(72) Erfinder: Glock, Gaston, 9220 Velden (AT)
(74) Vertreter: Patentanwälte Barger, Piso & Partner

(56) Entgegenhaltungen:
- WO-A1-2010/061355
- WO-A2-2007/054085
- ALEXANDER C FORD ET AL: "Efficacy of Biological Therapies in Inflammatory Bowel Disease: Systematic Review and Meta-Analysis", THE AMERICAN JOURNAL OF GASTROENTEROLOGY, Bd. 106, Nr. 4, 1. April 2011 (2011-04-01) , Seiten 644-659, XP055161736, ISSN: 0002-9270, DOI: 10.1038/ajg.2011.73

## Beschreibung

Die Erfindung betrifft die Behandlung entzündlicher Darmerkrankungen, auch IBD genannt, aus der englischen Abkürzung von inflammatory bowel diseases abgeleitet, darunter eitrige Dickdarmentzündung und Morbus Crohn, entsprechend dem Oberbegriff des Anspruchs 1.

Aus der WO 2010/061355 ist es "zur Behandlung verschiedener menschlicher krankhafter Zustände" bekannt, Klinoptilolith oral zu verabreichen. Dabei wird ein speziell vorbehandelter Klinoptilolith, der "potenzierter Klinoptilolith" genannt wird, verwendet. Unter den in der Beschreibung genannten zu behandelnden Krankheiten wird auch IBS genannt, eine Abkürzung für "irritable bowel syndrome", eine von IBD völlig verschiedene Erkrankung:

IBS ist eine Ausschlussdiagnose, die nicht direkt durch zielgerichtete Untersuchungen gestellt wird, sondern *indirekt* durch konsequentes Ausschließen anderer Krankheitsursachen, entsteht. Sie stellt quasi eine negative Schnittmenge aller möglichen Diagnosen dar, es handelt sich um eine "banale", prognostisch gutartige Erkrankung hoher Prävalenz, die nicht lebensbedrohlich oder lebensverkürzend ist.

IBD aber betrifft zwei schwere Krankheitsbilder, Morbus Crohn und Colitis Ulcerosa, die unbehandelt potentiell letal verlaufen und auch bei adäquater Behandlung die Lebenserwartung senken können. Die Diagnose beinhaltet in der Regel Rectosigmoidoskopie, Colonoskopie und/oder Gastroskopie und wird oftmals auch durch histologische Untersuchungen abgesichert.

Die in der WO für IBS dargelegte klinische Studie (in der ein Vorliegen von IBD anhand einer Rectosigmoidoskopie explizit ausgeschlossen wurde) lässt keinerlei Wirksamkeit bei IBD vorhersehen oder gar erwarten, schon auch weil es sich um medizinisch völlig verschiedene Krankheitsbilder handelt, die im Stand der Technik auch völlig unterschiedlich behandelt werden:
IBS mit Linaclotid,
IBD mit ASA, lokalen Glukokortikoiden wie Budenosid, monoklonalen Antikörpern (etwa gegen TNF wie Adalimumab und Infliximab oder gegen α4-ß7-Integrin wie Vedolizumab).

Übliche Behandlungen von IBD erfolgen, wie angeführt, mit Derivaten der 5-Aminosalicylsäure, mit systemisch oder lokal verabreichten Glucocorticoiden und mit Immunosuppressanten wie Azathioprin. Diese Medikamente führen jedoch zu einer Anzahl von schwerwiegenden, unangenehmen Nebeneffekten, sodass ihre Anwendung nur eingeschränkt erfolgen kann.

Es besteht somit ein Bedarf an einer Substanz oder Mittel, bzw. einem Medikament, beziehungsweise einer Kur, das bzw. die derartige Krankheiten ohne, oder zumindest mit verminderter, Belastung des Patienten durch Nebenwirkungen wirksam bekämpft.

Es ist Aufgabe der Erfindung, ein derartiges Medikament oder Mittel bzw. eine derartige Kur bzw. Behandlung anzugeben.

Dies wird durch die im kennzeichnenden Teil des Anspruches 1 angegebenen Merkmale erreicht. Mit anderen Worten, es wird, bevorzugt oral, ein Klinoptilolith zur Behandlung entzündlicher Darmerkrankungen, auch IBD genannt, verabreicht, der, bevorzugt, beispielsweise durch das Verfahren des Anmelders gemäß der EP 2 040 837, entsprechend der US 8,173,101, von Schwermetallen befreit ist, und der eine Partikelgröße zwischen 0,2 und 1 µm aufweist. Für Jurisdiktionen, in denen dies möglich oder gefordert ist, besteht die Erfindung darin, dass Klinoptilolith, der bevorzugt von Schwermetallen befreit ist, und der eine Partikelgröße zwischen 0,2 und 1 µm aufweist, zur Herstellung eines Medikamentes zur Behandlung der eingangs genannten Erkrankung(en) verwendet wird.

Klinoptilolith ist die Sammelbezeichnung für eine Gruppe von Mineralen aus der Gruppe der Zeolithe innerhalb der Mineralklasse der "Tektosilikate". Die Mineralgruppe kristallisiert monoklin und setzt sich in Form einer Mischkristallreihe aus sämtlichen Variationen folgender idealisierter Endglieder zusammen:
- Klinoptilolith-Ca: Ca₃(Si₃₀Al₆)O₇₂·20H₂O
- Klinoptilolith-K: K₆(Si₃₀Al₆)O₇₂·20H₂O
- Klinoptilolith-Na: Na₆(Si₃₀Al₆)O₇₂·20H₂O
Chemisch gesehen handelt es sich also um "wasserhaltige" Alumosilikate mit den im Kristallgitter eingebauten Kationen Calcium, Kalium bzw. Natrium.

Aufgrund ihres monoklinen Habitus, entwickeln Klinoptilolithe meist tafelige Kristalle, sie kommen aber auch in Form massiger Mineral-Aggregate vor. In reiner Form sind Klinoptilolith- Kristalle farblos und durchsichtig. Durch vielfache Lichtbrechung aufgrund von Gitterdefekten oder polykristalliner Ausbildung können sie aber auch weiß erscheinen oder durch Fremdeinschlüsse eine gelblichweiße bis rötlichweiße Farbe annehmen, wobei die Transparenz entsprechend abnimmt.

Klinoptilolithe, die in großen (oder abbauwürdigen) Mengen vorliegen sind zumeist Minerale sedimentären Ursprungs und gehen aus Ablagerungen vulkanischer Herkunft wie Tuffen und Vulkangläsern hervor. Daneben kann Klinoptilolith auch in Hohlräumen anderer magmatischer Gesteine wie Andesit, Basalt oder Rhyolith enthalten sein. Begleitende Minerale von Klinoptilolith sind weitere Zeolithe, Halit, Quarz, Calcit, Opal, Montmorillonit, Hectorit, Gaylussit, Thenardit und Seladonit. Wirtschaftlich ertragreiche Vorkommen befinden sich z.B. in Cornwall/England; Vogelsberg, Franken/Deutschland; Steiermark/Österreich; Andalusien/Spanien; Ionische Inseln/Griechenland; Ostküste Türkei; Honshu/Japan; südliches Neuseeland; Chubut/Argentinien; South Dakota, Wyoming sowie die Westküste der USA und Nova Scotia, Quebec, British Columbia/Kanada.

Dass Klinoptilolith in pharmazeutischen Anwendungen Verwendung findet, ist nicht nur aus der eingangs genannten Druckschrift bekannt (siehe Colella, C: A critical reconsideration of biomedical and veterinary applications of natural zeolites (2011). Clay Minerals 46: 295-309)

Die Erfindung wird im Folgenden unter Bezugnahme auf die Zeichnung näher erläutert. Dabei zeigt, bzw. zeigen
die Fig. 1 die Änderung des Körpergewichts über die Tage 0-10,
die Fig. 2 die Stuhlkonsistenz über die Tage 0-10,
die Fig. 3 und 4 die Länge des Dickdarms der einzelnen Gruppen,
die Fig. 5 histologische Darstellungen,
die Fig. 6 die Beeinträchtigungen des Dickdarms,
die Fig. 7 die Gesamtbeeinträchtigung,
die Fig. 8 bis 10 jeweils das Ergebnis verschiedener molekularbiologischer Untersuchungen und
die Fig. 11 die MPO Aktivität.

Die Wirksamkeit und die Unbedenklichkeit der erfindungsgemäßen Substanz gehen aus den nachfolgend geschilderten Untersuchungen und Experimenten hervor.

Es wurde für fünf Gruppen von je 12 Mäusen (Kontrolle nur 3 Mäuse) die im Zusammenhang mit der Behandlung von IBD häufig verwendete experimentelle Routine durchgeführt, bei der IBD durch Verabreichung von Dextransulfatnatrium (DSS) induziert wird. Dabei wurde DSS fünf Tage lang im Trinkwasser (ausgenommen bei der Kontrollgruppe) verabreicht, und sodann wurde die jeweilige Versuchssubstanz oral verabreicht. Am zehnten Tag wurden die Mäuse durch CO₂ getötet und der Dickdarm zur Überprüfung entnommen. Die einzelnen Gruppen waren dabei folgende:
Gruppe 1: Kontrollgruppe, erhielt kein DSS, nur 3 Mäuse, im Gegensatz von jeweils **12 Mäusen** in jeder anderen Gruppe.
Gruppe 2: erhielt ausschließlich DSS.
Gruppe 3: (GHC1) erhielt DSS wie oben beschrieben und ab dem sechsten Tag einen von Schwermetallen befreiten Klinoptilolith, der auf eine mittlere Partikelgröße von 3,5 µm vermahlen, somit nicht erfindungsgemäß, war.
Gruppe 4: (GHC2) erhielt ab dem sechsten Tag die erfindungsgemäße Substanz.
Gruppe 5: (5ASA) erhielt ab dem sechsten Tag Sulfasalazin, ein Derivat des Mesalazins, auch 5-ASA genannt.

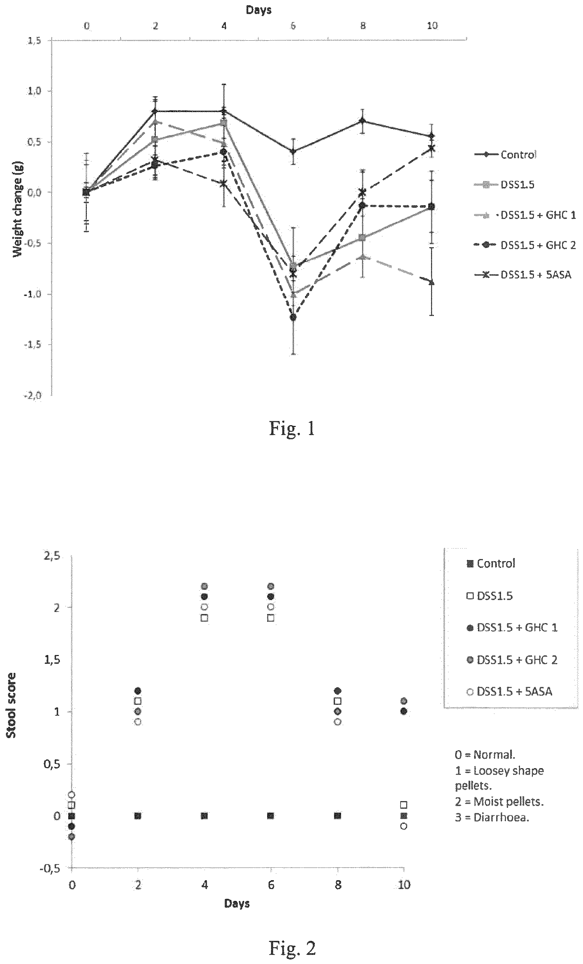

Die Resultate sind, zusammengefasst, die Folgenden: Der Gewichtsverlauf ist aus Fig. 1 ersichtlich. In dieser Figur steht:
Days für Tage;
Weight change (g) für Gewichtsänderung (g);
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5.

Aus dem Verlauf geht klar hervor, dass die nicht mit DSS behandelte Kontrollgruppe, insbesondere am sechsten Tag, deutlich größeres Gewicht aufwies, als alle mit DSS behandelten Gruppen.

Auffällig ist, dass die Behandlung mit nicht erfindungsgemäßem Klinoptilolith (GHC 1, Gruppe 3) die Gewichtszunahme nach der Verabreichung in der Heilungsphase eher zu behindern als zu unterstützen scheint, während dies bei der erfindungsgemäßen Substanz (GHC 2, Gruppe 3) nicht der Fall ist. Die erfindungsgemäße Substanz schneidet nur etwas schlechter ab als die Kontrollgruppe mit 5-ASA.

Ein weiteres Merkmal betreffend die Wirksamkeit der verabreichten Mittel ist die Stuhlkonsistenz, dargestellt in Fig. 2. Dabei steht
Stool score für Stuhlkonsistenz;
Days für Tage;
Loosely shape pellets für locker geformte Pellets;
Moist pellets für feuchte Pellets;
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5.

Erwartungsgemäß ändert sich bei der Kontrollgruppe nichts; die mit Klinoptilolith (egal welcher Korngröße) und die mit 5-ASA behandelten Gruppen waren am zehnten Tag wieder vollständig im normalen Bereich. Die Stuhlkonsistenz wird durch die Substanzeigenschaften der beiden Testsubstanzen (feines Pulver) dergestalt verändert, dass der Score auch nach 10 Tagen nicht 0 sondern ca. 1 beträgt. Der Verlauf zeigt jedoch klar, dass sich die Stuhlkonsistenz vom Tag 6 (Score > 2,0) auf Tag 10 (Score 1,0) deutlich verbessert. Die erfindungsgemäße Substanz kann Wasser binden und deshalb kann es dazu kommen, dass der Kot insgesamt weicher wird. Ein Wert von 1 zeigt jedoch eine relativ geringe Änderung, man kann demnach nicht wirklich von einer Persistenz der Krankheitssymptome sprechen.

Die Anwesenheit von Blut im Stuhl ist in nachfolgender Tabelle I angegeben. Dabei steht
Blood in stool für Blut im Stuhl;
Mice group für Mäusegruppe;
Days für Tage;
Control für Kontrollgruppe;
DSS 1.5 für die Gruppe 2;

DSS1.5+GHC1 für die Gruppe 3;
DSS1.5+GHC2 für die (erfindungsgemäße) Gruppe 4;
DSS1.5+5ASA für die Gruppe 5.

Wie ersichtlich ist, war lediglich in der mit DSS behandelte Gruppe vom sechsten bis zum zehnten Tag Blut im Stuhl nachweisbar (+). Die beiden mit Klinoptilolith behandelten Gruppen (3 und 4) zeigten vom sechsten bis zum achten Tag, und die mit 5-ASA behandelte Gruppe nur am sechsten Tag Blut im Stuhl.

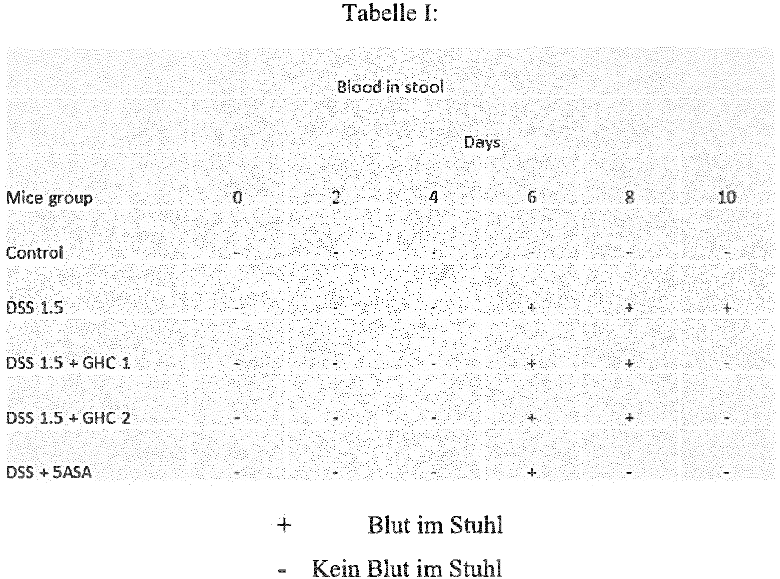

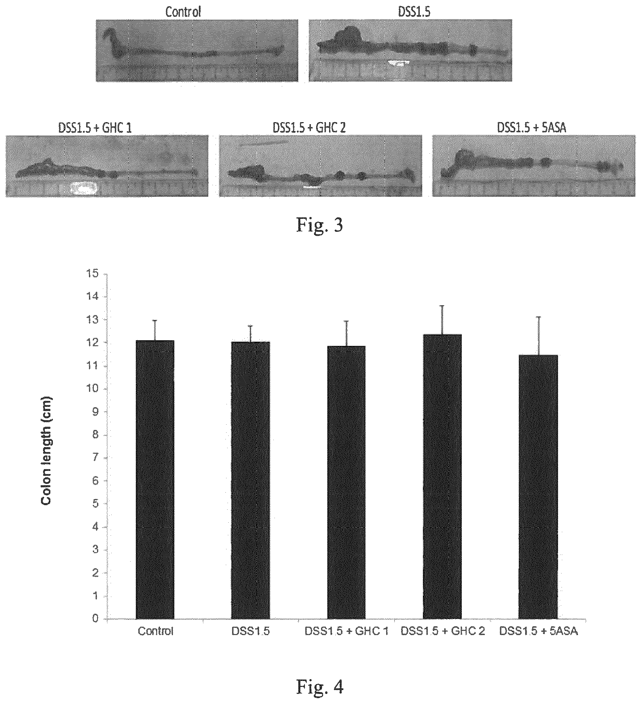

Die nach dem Töten der Tiere festgestellten Veränderungen an der Dickdarmlänge sind aus den fünf Darstellungen der Fig. 3 qualitativ ersichtlich. Die Abbildungen zeigen keine markanten Unterschiede zwischen den einzelnen Gruppen. Eine quantitative Auswertung der Dickdarmlänge der einzelnen Gruppen ist in Fig. 4 gezeigt (jeweils gemittelt). Dabei steht in beiden Fällen
Control für Kontrollgruppe;
Colon lenght (cm) für Länge des Dickdarms (cm);
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5.

Eine zunehmende Verkürzung des Darms zeigt den zunehmenden Grad der Entzündung an. Ein (statistisch nicht signifikanter) Trend der erfindungsgemäßen Substanz, gegenüber allen anderen Gruppen, einen längeren Dickdarm zu fördern, ist aus Fig. 4 zu erkennen.

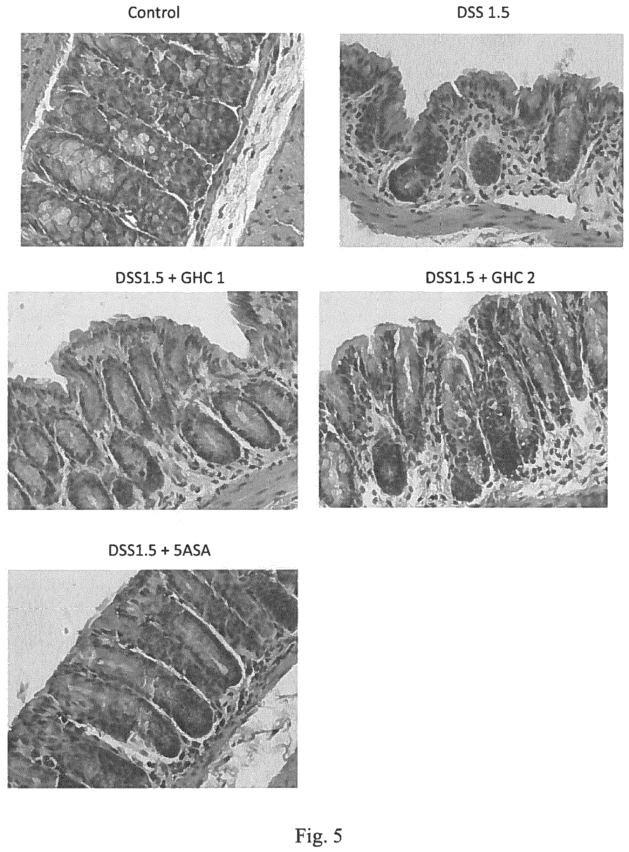

Die Fig. 5 zeigt eine histologische Analyse, nämlich eine repräsentative Darstellung der Hämatoxylin-Eosin-Färbung jeder Versuchsgruppe. Dabei steht
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5.

Die Darstellungen zeigen ein unbeschädigtes Darmepithel bei der Kontrollgruppe (Gruppe 1). Im Gegensatz dazu ist eine verbreitete Entzündung bei der Gruppe 2 zu sehen (Negativkontrolle mit DSS). Durch die Behandlung mit Klinoptilolith (egal welcher Korngröße) konnte man die Heilung des Darmepithel teilweise beschleunigen (Gruppen 3 und 4). Die Positivkontrollgruppe (Gruppe 5) zeigt eine deutlich verbesserte Heilung des Darmepithels mit nur spärlichen Zeichen von Epithelbeschädigung und Immunzellen- Infiltration.

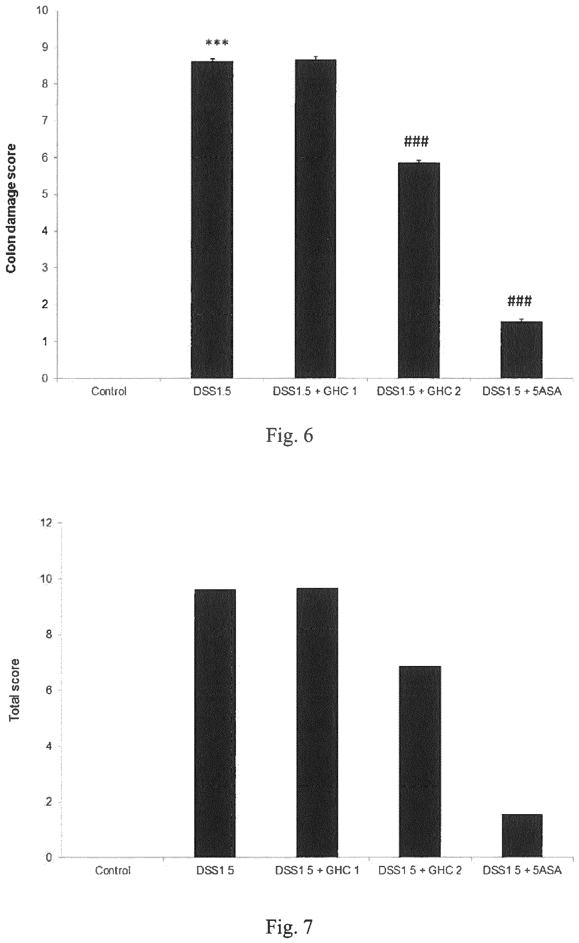

Die Fig. 6 zeigt den "colon damage score", eine Bestimmung der Darmschädigung anhand histo(patho)logischer Untersuchungen. Der "colon damage score" ist von (a) Zerstörung des Epithels (Skala 0 bis 3) (b) Ausdehnung der Krypten (Skala 0 bis 3) (c) Abwesenheit von Becherzellen (Skala 0 bis 3) (d) Infiltration von Immunzellen (Skala 0 bis 3) (e) Verteilung der entzündlichen Zellen (Skala 0 bis 3) abgeleitet, dabei steht
Control für die Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5;
Colon damage score für Bestimmung der Darmschädigung anhand histo(patho)-logischer Untersuchungen;

*** stellt eine höchst signifikante Differenz zur Kontrollgruppe (Control) dar;
### stellt eine höchst signifikante Differenz zur DDS1.5 Gruppe (Gruppe 2) dar.

Wiederum ist die gute Annäherung der 5-ASA (Gruppe 5) und der mit GHC2 (Gruppe 4) erfindungsgemäß behandelten Mäuse an die Kontrollgruppe zu sehen. Die Gruppe, welche mit normalem Klinoptilolith behandelt wurde (GHC1, Gruppe 3), ist hingegen statistisch nicht von der Gruppe ohne Behandlung (DSS 1,5, Gruppe 1) zu differenzieren.

Die Fig. 7 zeigt den "Total score", der ein Maß für den gesamten entzündlichen Zustand der Tiere darstellt. Der "total score" (auch Disease Activity Index genannt, siehe Cooper HS, Murthy SN, Shah RS, Sedergran DJ. "Clinicopathologic study of dextran sulfate sodium experimental murine colitis". Lab Invest 1993;69:238-249.) ist aus den Ergebnissen in Fig. 2 bis 5 sowie den Inhalten der Tabelle I abgeleitet. Die Skala der Fig. 6 ist demzufolge relativ und hat keine feste Einheit. Dabei steht
Total score für den gesamten entzündlichen Zustand;
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5.

Dieser Abbildung, die mit zunehmendem Wert (0 bis 12) zunehmende Schädigung anzeigt, ist zu entnehmen, dass, abgesehen von der Kontrollgruppe mit 5-ASA, die geringsten Änderungen (zusammengefasst im "Total score" = Summe der Parameter Stuhl, Darmlänge und Darmschädigungs- Score) im Vergleich zur Kontrollgruppe ohne Darmschädigung (Control) ausschließlich bei der Gruppe 4 zu finden waren, die die erfindungsgemäße Substanz erhielt. Die Gruppe 3, die normalen Klinoptilolith erhielt, zeigt keine solche Verbesserung im Vergleich zur Gruppe ohne Behandlung (DSS 1.5).

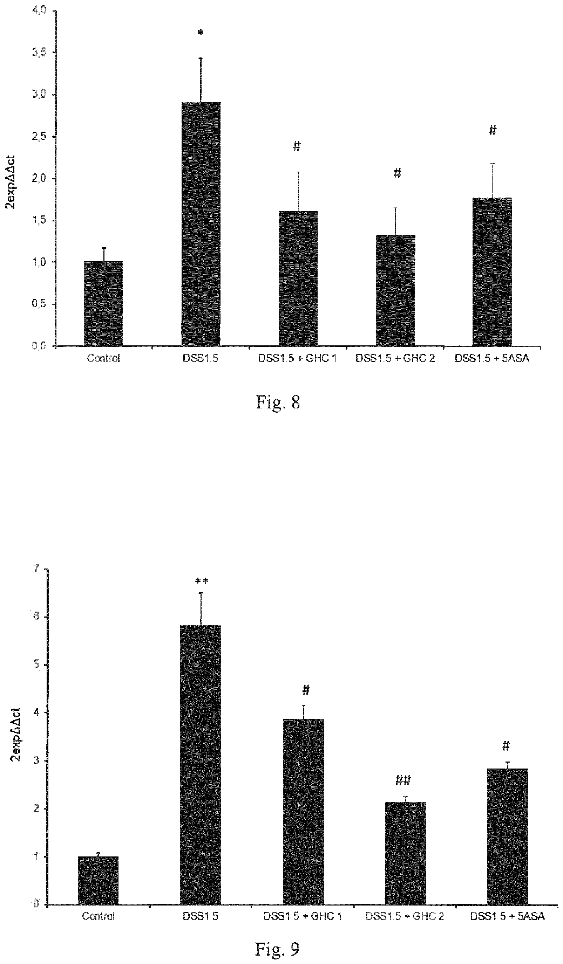

Zur Objektivierung und Quantifizierung der auf Basis der histologischen Untersuchungen ermittelten Effekte wurden weitergehenden Analysen biochemischer Entzündungsmarker (TNF-α/IL-6/IL-1β-mRNA mittels RT-PCR, MPO Aktivität) aus Gewebe- Homogenaten der relevanten Darmabschnitte durchgeführt. Die Ergebnisse für IL-1β sind in Fig. 8 dargestellt. Dabei steht
2expΔΔct für den Anstieg der Expression für ein bestimmtes Gen (1=1x, 2=2x usw.);
Control für Kontrollgruppe;
DSS1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5;
* stellt eine signifikante Differenz mit der Kontrollgruppe (Control) dar;
# stellt eine signifikante Differenz mit der DDS1.5 Gruppe (Gruppe 2) dar.

Die Neusynthese von IL-1β ist in Fig. 8 dargestellt. IL-1β ist eine pro-entzündliches Zytokin, dessen Senkung eine antientzündliche Wirkung zeigt. Wiederum ist eine bessere Wirkung bei der Versuchsgruppe mit Applikation der erfindungsgemäßen Substanz (GHC 2) als bei Behandlung mit normalem Klinoptilolith (GHC 1) nachweisbar.

Zwei weitere, pro-entzündliche Zytokine wurden untersucht, die Ergebnisse sind in Fig. 9 (IL-6) und 10 (TNF-α) dargestellt. Dabei steht
2expΔΔct für den Anstieg der Expression für ein bestimmtes Gen (1=1x, 2=2x usw.);
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5;

** stellt eine sehr signifikante Differenz zur Kontrollgruppe (Control) dar;
# stellt eine signifikante Differenz zur DDS 1.5 Gruppe (Gruppe 2) dar;
## stellt eine sehr signifikante Differenz zur DDS1.5 Gruppe (Gruppe 2) dar.

Bei I1-6 (Fig. 9) zeigt wiederum ausschließlich die erfindungsgemäße Substanz (Gruppe 4) eine stärkere Wirkung als die Behandlung mit normalem Klinoptilolith (GHC 1), sie ist sogar besser als die Kontrolle mit der Standardbehandlung mittels 5-ASA.

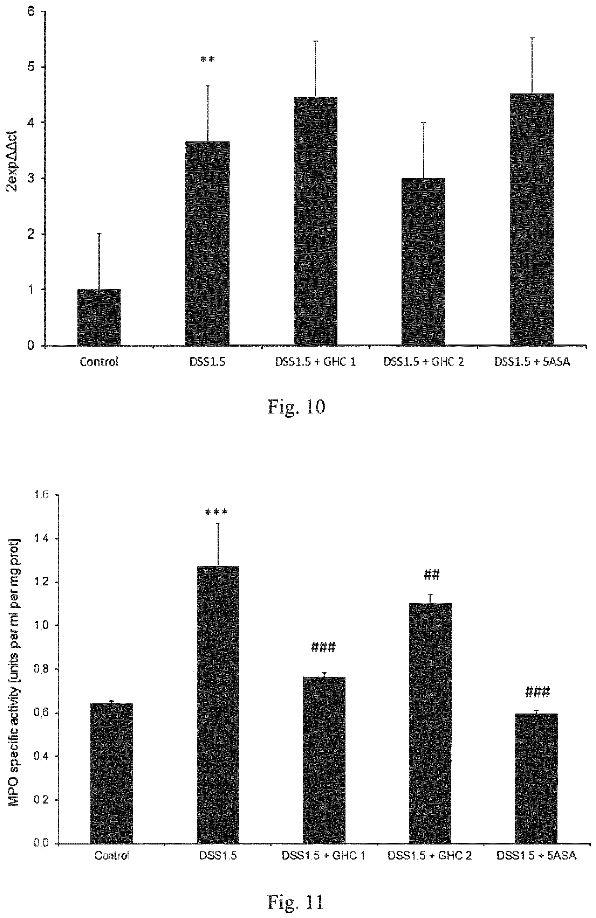

Fig. 10 zeigt die Ergebnisse für TNF-α, dabei ist für die erfindungsgemäße Substanz kein statistisch signifikanter Unterschied zur Kontrolle ohne Behandlung (+) nachweisbar. Da auch die Behandlung mit 5-ASA keinen positiven Effekt auf TNF-α zeigt, ist die Aussagekraft dieses Parameters zur Beurteilung des Heilungsfortschrittes in Frage zu stellen.

Schließlich wurde auch noch die MPO Aktivität im Darmgewebe gemessen, die Ergebnisse sind in Fig. 11 dargestellt. Dabei steht
Control für Kontrollgruppe;
DSS 1.5 für die Gruppe 2;
DSS1.5+GHC1 für die Gruppe 3;
DSS1.5+GHC2 für die (erfindungsgemäße) Gruppe 4;
DSS1.5+5ASA für die Gruppe 5;
MPO specific activity [units per ml per mg prot] für spezifische MPO Aktivität [Einheiten pro ml, normalisiert auf den Proteingehalt];
*** stellt eine höchst signifikante Differenz zur Kontrollgruppe (Control) dar;
## stellt eine sehr signifikante Differenz zur DDS1.5 Gruppe (Gruppe 2) dar;
### stellt eine höchst signifikante Differenz zur DDS1.5 Gruppe (Gruppe 2) dar.

Die MPO Aktivität ist eine übliche Messung in CED Studien (Kim JJ, Shajib MS, Manocha MM, Khan WI. "Investigating intestinal inflammation in DSS-induced model of IBD" J Vis Exp. 2012 Feb 1;(60)), sie stellt die Entzündung des Darms durch die Quantifizierung der von infiltrierten Neutrophilen produzierten Myeloperoxidase dar. Die Ergebnisse (Fig. 11) zeigen eine Wirkung der erfindungsgemäßen Substanz wie auch der bei Behandlung mit normalem Klinoptilolith (Gruppe 3). Offensichtlich beruht der Einfluss auf diesen Parameter auf einem anderen Wirkmechanismus, der die Wirkung der erfindungsgemäßen Substanz nicht entsprechend wiedergeben kann.

Die Experimente zeigen auch, dass die Tiere Klinoptilolith sowohl in der Korngröße der Gruppe 3, als auch den erfindungsgemäß feiner zerteilten (Gruppe 4), gut vertragen und dessen Verabreichung keine Probleme bereitet. Es kam zu keinen durch die Applikation der beiden Testsubstanzen verursachten Todesfällen oder sonstigen Anzeichen einer Toxizität.

Die Verabreichung des erfindungsgemäßen Klinoptiloliths erfolgt bevorzugt oral, allein oder mit in der Medizin üblichen Trägersubstanzen oder Füllstoffen (Laktose, Glucose, Saccharose, Stärke, Calciumsulfat oder mikrokristalline Cellulose).

Unter "Klinoptilolith mit einer Partikelgröße zwischen 0,2 und 1 µm" wird verstanden, dass Klinoptilolith dieser Partikelgröße in wirksamer Menge vorliegt. Klinoptilolith mit geringerer Partikelgröße, der schon aufgrund des Mahlvorganges und trotz sorgfältiger Siebung unvermeidlich enthalten ist bzw. vorliegt, ebenso wie solcher mit größerer Partikelgröße, sofern vorhanden, wird nicht gezählt bzw. nicht beachtet. Erhalten wird der erfindungsgemäß verwendete bzw. verwendbare Klinoptilolith durch Mahlen und Sieben. Die Herkunft des Klinoptiloliths spielt keine merkliche Rolle.

Dass die Korngröße des Klinoptiloliths einen deutlichen Einfluss auf seine Wirksamkeit hat, geht aus dem Vergleich der Resultate der Gruppe 3 (nicht erfindungsgemäß) mit den Resultaten der Gruppe 4 (erfindungsgemäß) deutlich hervor. Die Korngrößenverteilung innerhalb der Grenzen hat keinen signifikanten Einfluss auf die Wirksamkeit.

Bestimmt wird diese Korngröße beispielsweise durch Sedimentation oder durch Auswertung der Streuung von Laserlicht, oder durch digitale Bildverarbeitung, wie im Stand der Technik zur Genüge bekannt.

Die Verabreichungsdauer wird in Abhängigkeit vom Remissionsverlauf durch den behandelnden Arzt individual an den Patienten angepasst. Gleiches gilt auch für die zu verabreichende Dosis, die sich im Bereich zwischen 1 bis 500 mg/kg, bevorzugt zwischen 5 und 100 mg/kg Körpergewicht bewegt.

Bei oraler Verabreichung wird der erfindungsgemäße Klinoptilolith gegebenenfalls mit pharmazeutisch unbedenklichen Trägermaterialien und/oder Verdünnungsmitteln versetzt. Diese sind beispielsweise: Laktose, Glucose, Saccharose, Stärke, Calciumsulfat, mikrokristalline Cellulose und viele andere im Stand der Technik bekannte und verwendete Substanzen.

### Allgemein-mathematisches zur Beurteilung:

Unter Signifikanz in der analytischen Statistik versteht man die Wahrscheinlichkeit, dass ein Merkmal bzw. eine Ausprägung einer Stichprobe mit der Gesamtpopulation übereinstimmt. Diese Wahrscheinlichkeit wird durch den p-Wert berechnet. Der p-Wert gibt dabei an, in wie viel % aller Fälle das tatsächliche Endergebnis der Gesamtpopulation außerhalb des errechneten Bereichs liegen wird. Je nach Ergebnis des p-Werts können diese zusätzlich eingestuft und interpretiert werden:
"Höchst Signifikant" p ≤ 0,001 weniger als 0,1% aller Fälle des tatsächlichen Gesamtergebnisses liegen außerhalb des betrachteten Bereichs.
"Sehr signifikant p ≤ 0,01 weniger als 1% aller Fälle des tatsächlichen Gesamtergebnisses liegen außerhalb des betrachteten Bereichs.
"Signifikant" p ≤ 0,05 weniger als 5% aller Fälle des tatsächlichen Gesamtergebnisses liegen außerhalb des betrachteten Bereichs.

## Patentansprüche

1. Klinoptilolith, der eine Partikelgröße zwischen 0,2 und 1 µm aufweist, zur Verwendung bei der Behandlung von entzündlichen Darmerkrankungen, auch IBD (Inflammatory Bowel Diseases) genannt, von Säugetieren und Menschen.

2. Klinoptilolith zur Verwendung nach Anspruch 1, **dadurch gekennzeichnet, dass** er von Schwermetallen befreit ist.

3. Klinoptilolith zur Verwendung nach Anspruch 1 oder 2, **dadurch gekennzeichnet, dass** er, gegebenenfalls mit pharmazeutisch unbedenklichen Trägermaterialien und/oder Verdünnungsmitteln, oral verabreicht wird.

4. Klinoptilolith zur Verwendung nach Anspruch 3, **dadurch gekennzeichnet, dass** die zu verabreichende Dosis im Bereich zwischen 1 bis 500 mg/kg, bevorzugt zwischen 5 und 100 mg/kg Körpergewicht des zu behandelnden Säugetiers bzw der zu behandelnden Person liegt.

## Claims

1. Clinoptilolite having a particle size between 0.2 and 1 µm for use in the treatment of inflammatory bowel disease, also termed IBD, in mammals and humans.

2. Clinoptilolite according to Claim 1, **characterized in that** it is freed of heavy metals.

3. Clinoptilolite according to Claim 1 or 2, **characterized in that** it is administered orally, optionally with pharmaceutically harmless carrier materials and/or diluents.

4. Clinoptilolite according to Claim 3, **characterized in that** the dose to be administered is within the range between 1 to 500 mg/kg, preferably between 5 and 100 mg/kg, of body weight of the mammal to be treated or of the person to be treated.

## Revendications

1. Clinoptilolite, qui présente une taille de particule comprise entre 0,2 et 1 µm, destinée à l'utilisation dans le traitement de maladies intestinales inflammatoires, également dénommées IBD (Inflammatory Bowel Diseases), des mammifères et êtres humains.

2. Clinoptilolite destinée à l'utilisation selon la revendication 1, **caractérisée en ce qu'**elle est privée de métaux lourds.

3. Clinoptilolite destinée à l'utilisation selon la revendication 1 ou 2, **caractérisée en ce qu'**elle est administrée par voie orale, éventuellement avec des substances véhicule et/ou diluants pharmaceutiquement acceptables.

4. Clinoptilolite destinée à l'utilisation selon la revendication 3, **caractérisée en ce que** la dose à administrer se situe dans la plage comprise entre 1 et 500 mg/kg, de préférence entre 5 et 100 mg/kg de poids corporel du mammifère à traiter ou de la personne à traiter.
